(19) Europäisches Patentamt
European Patent Office
Office européen des brevets

(11) EP 3 685 844 A1

(12) EUROPEAN PATENT APPLICATION
published in accordance with Art. 153(4) EPC

(43) Date of publication:
29.07.2020 Bulletin 2020/31

(51) Int Cl.:
A61K 38/01 (2006.01)
A61K 38/16 (2006.01)
A23L 33/19 (2016.01)
A61P 3/00 (2006.01)

(21) Application number: 18858320.7

(22) Date of filing: 23.03.2018

(86) International application number:
PCT/JP2018/011811

(87) International publication number:
WO 2019/058609 (28.03.2019 Gazette 2019/13)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 19.09.2017 JP 2017179140

(71) Applicant: Morinaga Milk Industry Co., Ltd.
Minato-ku
Tokyo 108-8384 (JP)

(72) Inventors:
• MATSUNAGA, Yutaka
Zama-shi
Kanagawa 252-8583 (JP)
• SAKATA, Yasuyuki
Zama-shi
Kanagawa 252-8583 (JP)
• YAGO, Takumi
Zama-shi
Kanagawa 252-8583 (JP)

(74) Representative: Vossius & Partner
Patentanwälte Rechtsanwälte mbB
Siebertstrasse 3
81675 München (DE)

(54) COMPOSITION FOR PROMOTING ENERGY CONSUMPTION

(57) Provided is a technique that allows for the promotion of energy expenditure. The present invention is a composition for promoting energy expenditure, including, as an active ingredient, a milk protein hydrolysate having an average molecular weight of 220 Daltons or more and 1,000 Daltons or less, a peptide having only Met-Lys-Pro, or a peptide including the amino acid sequence.

FIG. 1

## Description

Technical Field

The present invention relates to an energy expenditure promoting agent and also to a composition for promoting energy expenditure. The energy expenditure promoting agent and composition for promoting energy expenditure can be utilized as pharmaceuticals, foods, beverages, or animal feeds.

Background Art

In recent years, together with an increase in lipid intake due to the westernization of eating habits, obesity due to the lack of exercise or the like has become a problem. Obesity is a condition in which excess body fat has accumulated to cause a person to be overweight, and serves as a risk factor that causes lifestyle-related diseases such as diabetes.

It is thought that obesity occurs when the balance between energy intake and expenditure is disrupted, and intake exceeds expenditure. For preventing or alleviating metabolic syndrome including obesity, it is considered effective to do more physical activity to thereby increase energy expenditure.

Energy expenditure can be mainly divided, in addition to basal metabolism, into diet-induced thermogenesis resulting from meals and thermogenesis resulting from physical activities. Thermogenesis resulting from physical activities includes thermogenesis due to exercise, such as sports, and non-exercise activity thermogenesis (Non Exercise Activity Thermogenesis: NEAT) resulting from other routine physical activities (NPL 1). Furthermore, it is known that energy expenditure by exercise does not only occur during exercise, but energy expenditure continuously increases even after the completion of exercise.

As a component that promotes energy expenditure, caffeine having an activating action on the sympathetic nervous system, for example, has been reported in the past (NPL 2). However, meanwhile, a possibility that the ingestion of caffeine may cause an increase in blood pressure has been reported (NPL 3), and, in terms of safety and overstimulation, its practicality is limited.

Incidentally, a casein hydrolysate obtained by hydrolyzing a casein from milk or the like is known to have a blood cholesterol or blood neutral fat reducing action, a lipid metabolism improving action, and the like (PTLs 1 to 3).

In addition, it has been reported that a casein hydrolysate having about 50 mass% free amino acids increases lipid utilization, but does not affect energy expenditure (NPL 4).

In addition, it is also known that a tripeptide having only the amino acid sequence Met-Lys-Pro contained in a casein hydrolysate has an angiotensin-converting enzyme inhibiting action and a bradykinin inactivation suppressing action, and is effective in reducing blood pressure (PTL 4).

In addition, it is known that a whey protein hydrolysate obtained by hydrolyzing a whey protein has a gastrointestinal hormone GLP-2 secretion promoting action, a cysteine protease inhibiting action, an interleukin-18 inducing action, and the like (PTLs 5 to 7).

Citation List

Patent Literature

PTL 1: Japanese Patent No. 3920933
PTL 2: Japanese Patent No. 5972235
PTL 3: WO 2007/142230
PTL 4: WO 2003/044044
PTL 5: Japanese Patent No. 4219707
PTL 6: JP-A-2005-139084
PTL 7: JP-A-2009-137982

Non Patent Literature

NPL 1: Levine JA, J. Intern. Med., 2007, 262 (3) 273-287
NPL 2: Dulooo AG. et al., Am. J. Clin. Nutr. 1989, 49 (1) 44-50
NPL 3: Nurminen ML. et al., Eur. J. Clin Nutr. 1999, 53 (11) 831-9

NPL 4 : Lillefosse et al., J. Nutrition, 2013, 143 (9) 1367-1375

Summary of Invention

Technical Problem

An object of the invention is to provide a technique that allows for the promotion of energy expenditure.

Solution to Problem

The present inventors have conducted extensive research to solve the above problems. As a result, they have found that a specific milk protein hydrolysate has an excellent energy expenditure promoting action, and thus accomplished the invention.
That is, the invention is an energy expenditure promoting agent or a composition for promoting energy expenditure, containing a milk protein hydrolysate having an average molecular weight of 220 Daltons or more and 1,000 Daltons or less as an active ingredient (hereinafter also referred to as "composition for promoting energy expenditure of the invention"). Incidentally, in the invention, the terms "composition" and "agent" have the same meaning.
In one mode of the invention, the composition for promoting energy expenditure is preferably used for raising a body temperature.
In addition, in one mode of the invention, it is preferable that the composition for promoting energy expenditure is ingested after exercise.
In addition, in one mode of the invention, it is preferable that the milk protein is a casein or a whey protein.
In addition, in a preferred mode of the invention, the milk protein hydrolysate is a hydrolysate by a proteolytic enzyme.
In addition, in one mode of the invention, it is preferable that the milk protein hydrolysate contains at least a peptide having only Met-Lys-Pro.
In addition, in a preferred mode of the invention, the energy expenditure is attributable to a factor selected from the group consisting of basal metabolism, meal-induced thermogenesis, non-exercise activity thermogenesis, and thermogenesis due to exercise.
In addition, in a preferred mode, the composition for promoting energy expenditure of the invention is a pharmaceutical for treating and/or preventing impaired energy expenditure or for promoting energy expenditure.
In addition, another mode of the invention is a food or beverage composition for promoting energy expenditure, containing a milk protein hydrolysate having an average molecular weight of 220 Daltons or more and 1,000 Daltons or less as an active ingredient.
In addition, the food or beverage composition of this mode is preferably used for raising a body temperature.
In addition, it is preferable that the food or beverage composition of this mode is ingested after exercise.
In addition, in the food or beverage composition of this mode, it is preferable that the milk protein is a casein or a whey protein.
In this mode, the milk protein hydrolysate is preferably a hydrolysate by a proteolytic enzyme.
In addition, in this mode, it is preferable that the milk protein hydrolysate contains at least a peptide having only or including the sequence Met-Lys-Pro (hereinafter also referred to as "MKP peptide"). Incidentally, the peptide including Met-Lys-Pro refers to a peptide that includes the sequence Met-Lys-Pro and has one or a plurality of arbitrary amino acids added on the C-terminal and/or N-terminal side thereof. Here, "a plurality of" is preferably 2 to 10, but is not particularly limited thereto.
In this mode, the energy expenditure is preferably attributable to a factor selected from the group consisting of basal metabolism, meal-induced thermogenesis, non-exercise activity thermogenesis, and thermogenesis due to exercise.

Advantageous Effects of Invention

According to the invention, a highly safe composition for promoting energy expenditure is provided. The composition for promoting energy expenditure of the invention can be contained in a pharmaceutical or a food or beverage composition, and is effective in treating, alleviating, and/or preventing impaired energy expenditure, promoting energy expenditure, and improving and/or increasing the energy expenditure after exercise.

Brief Description of Drawings

[Fig. 1] Fig. 1 is a graph showing time-dependent changes in energy expenditure when a casein hydrolysate was ingested after exercise.

[Fig. 2] Fig. 2 is a graph showing time-dependent changes in energy expenditure when a casein hydrolysate was ingested at rest.

[Fig. 3] Fig. 3 is a graph showing energy expenditure for 60 minutes after the ingestion of a casein hydrolysate, a whey protein hydrolysate, or a MKP peptide at rest.

Description of Embodiments

Next, the invention will be described in detail. However, the invention is not limited to following embodiments, and can be freely changed within the scope of the invention.

<Composition for Promoting Energy Expenditure>

The energy expenditure promoting agent or composition for promoting energy expenditure of the invention contains a milk protein hydrolysate having an average molecular weight of 220 Daltons or more and 1,000 Daltons or less as an active ingredient.

The average molecular weight of the milk protein hydrolysate in the invention is 220 Daltons (hereinafter "Da") or more and 1, 000 Da or less as described above, but is preferably 250 Da or more and 400 Da or less, and more preferably 300 Da or more and 390 Da or less.

Incidentally, in the invention, when a hydrolysate of a casein is utilized, it is more preferable that the average molecular weight is 350 to 390 Da, while in the case of a whey protein, it is preferable that the average molecular weight is 360 to 400 Da.

Incidentally, the average molecular weight of the casein hydrolysate of NPL 4 is estimated to be lower than in the invention.

The average molecular weight of a milk protein hydrolysate in the invention is determined based on the concept of the number average molecular weight described below.

As described in a literature (edited by the Society of Polymer Science, Japan, "Kobunshi Kagaku No Kiso (Foundation of Polymer Science)", pp. 116 to 119, Tokyo Kagaku Dojin, 1978), for example, the number average molecular weight (Number Average of Molecular Weight) indicates the average molecular weight of a polymer compound based on a different index as follows.

That is, a polymer compound, such as a protein hydrolysate, is a heterogeneous substance, and the molecular weight has a distribution. Therefore, for physicochemical handling, the molecular weight of a protein hydrolysate needs to be expressed as an average molecular weight. The number average molecular weight (hereinafter sometimes abbreviated to Mn) is an average over the number of molecules . When the molecular weight of the peptide chain i is Mi, and the number of molecules is Ni, Mn can be defined by the following formula.

[Equation 1]

$$Mn = \sum_{i=1}^{\infty} Mi\, Ni \Big/ \sum_{i=1}^{\infty} Ni$$

As used herein, the average molecular weight of a milk protein hydrolysate refers to a value measured and calculated by the following method. That is, by means of high-performance liquid chromatography, using a polyhydroxyethyl aspartamide column (manufactured by Poly LC; 4.6 in diameter $\times$ 200 mm), elution is performed with 20 mM sodium chloride and 50 mM formic acid at an elution rate of 0.4 mL/min (edited by Nobuo UI et al., "Tanpakushitsu/Pepuchido No Kosokuekitai Kurmatogurafi (High-Performance Liquid Chromatography of Protein/Peptide)", Chemistry Extra No. 102, p. 241, Kagaku Dojin, 1984). Detection is performed using a UV detector (manufactured by Shimadzu Corporation), and the data is analyzed by a GPC analysis system (manufactured by Shimadzu Corporation) to calculate the number average molecular weight. Incidentally, as a reference sample for the molecular weight calculation, a protein and/or peptide whose molecular weight is known may be suitably used.

A protein hydrolysate generally contains free amino acids in the course of production. In the invention, the percentage of free amino acids in the milk protein hydrolysate is preferably 40 mass% or less, more preferably 35 mass% or less, still more preferably 15 mass% or less, and particularly preferably 10 mass% or less.

As used herein, the percentage of free amino acids is the proportion of the free amino acid content in the entire milk protein hydrolysate, and can be measured and calculated by the following method.

For amino acids other than tryptophan, cysteine, and methionine, a sample is hydrolyzed with 6-N hydrochloric acid at 110°C for 24 hours. For tryptophan, a sample is alkali-decomposed with barium hydroxide at 110°C for 22 hours. For cysteine and methionine, a sample is treated with performic acid and then hydrolyzed with 6N hydrochloric acid at 110°C for 18 hours. Then, each is analyzed by an automatic amino acid analyzer (manufactured by Hitachi, Model 835) to measure the mass of amino acids. The composition of each amino acid in the sample is measured by the above method, and the results are summed to calculate the total mass of amino acids in the sample. Subsequently, the sample is deproteinized with sulfosalicylic acid, the masses of residual free amino acids are each measured by the above method, and the results are summed to calculate the total mass of free amino acids in the sample. From these values, the content of free amino acids in the sample is calculated by the following formula:

```
Percentage of free amino acids (mass%) = (total mass of free amino acids/total mass of amino acids) × 100
```

The above milk protein hydrolysate can be preferably obtained usually by subjecting a milk protein to a decomposition treatment, preferably to hydrolysis. The milk protein decomposition treatment is not particularly limited, and may be an enzyme treatment, an acid treatment, an alkali treatment, a heat treatment, or the like. It is also possible to suitably combine two or more of these treatments .

The milk protein hydrolysate herein is not particularly limited, but is preferably a casein hydrolysate or a whey protein hydrolysate.

The casein to serve as a starting material in obtaining a casein hydrolysate may be, for example, one kind or a mixture of two or more kinds selected from various commercially available caseins; micellar casein; acid caseins such as lactic acid casein and hydrochloric acid casein; caseinates such as sodium caseinate, potassium caseinate, and calcium caseinate; and the like. The mixture may be a mixture of any proportions.

The whey protein to serve as a starting material in obtaining a whey protein hydrolysate is not particularly limited, and preferred examples thereof include various commercially available whey proteins, such as whey protein concentrates (WPC) and whey protein isolates (WPI). In addition, the whey protein can also be purified in the usual manner from cow milk, defatted milk, whole milk powder, or skim milk powder.

In addition, the casein or whey protein is preferably a casein or whey protein isolated by a known method from cow milk, defatted milk, whole milk powder, skim milk powder, or the like.

Hereinafter, a preferred method for obtaining a milk protein hydrolysate will be described, but the method is not particularly limited thereto.

A raw material milk protein is dispersed in water or warm water and dissolved to prepare an aqueous milk protein solution. The concentration of the aqueous milk protein solution is not particularly limited, but is preferably set to make a protein concentration usually within a range of 2 mass% or more, still more preferably about 5 to 15 mass%.

Further, it is preferable that the aqueous milk protein solution is desalted by an ion exchange method using a sodium-form or potassium-form cation exchange resin (preferably a strongly acidic cation exchange resin), an electrodialysis method, an ultrafiltration membrane method, a loose reverse osmosis membrane method, or the like to suitably perform pH adjustment or calcium concentration adjustment. At the time of desalting, either of a column process and a batch process may be employed. In addition, the aqueous milk protein solution may be suitably heat-sterilized before desalting, for example.

Subsequently, the aqueous milk protein solution is subjected to a hydrolysis treatment. The hydrolysis treatment may be an enzyme treatment, an acid treatment, an alkali treatment, a heat treatment, or the like, and it is also possible to suitably combine two or more of these treatments.

Examples of proteolytic enzymes of the present disclosure include those from plants, animals, microorganisms, and the like. They may be used alone, and it is also possible to use a combination of two or more kinds. As the proteolytic enzyme, an endoprotease is preferable.

Examples of such endoproteases include serine protease, metallo protease, cysteine protease, and aspartic proteinase. They may be used alone, and it is also possible to select and use two or more kinds. Among these, it is preferable to use serine protease and/or metallo protease.

In addition, proteases are classified into alkaline proteases, neutral proteases, and acidic proteases. Among them, it is preferable to use neutral proteases.

As the proteolytic enzyme, a commercially available product can be used. Examples of such proteolytic enzymes include Bioprase (manufactured by Nagase Biochemicals, Ltd.), Proleather (manufactured by Amano Enzyme Inc.), Protease S (manufactured by Amano Enzyme Inc.), PTN6.0S (manufactured by Novozymes), Savinase (manufactured by Novozymes), GODO B.A. P (manufactured by Godo Shusei Co., Ltd.), Protease N (manufactured by Amano Enzyme Inc.), GODO B.N.P (manufactured by Godo Shusei Co., Ltd.), Neutrase (manufactured by Novozymes), Alcalase (manufactured by Novozymes), Trypsin (manufactured by Novozymes), Chymotrypsin (manufactured by Novozymes), Subtilisin (manufactured by Novozymes), Papain (manufactured by Amano Enzyme Inc.), and Bromelain (manufactured by Amano Enzyme Inc.) . These enzymes may be used alone, and it is also possible to select and use two or more kinds.

Among these, it is preferable to use one kind or two or more kinds of a neutral protease selected from subtilisin (e.g., Bioprase), trypsin (e.g., PTN6.0S), and bacillolysin (e.g., Protease N), and it is more preferable to use a combination of these three kinds.

The amount of endoprotease used relative to the milk protein is not particularly limited, and may be suitably adjusted according to the substrate concentration, the enzyme titer, the reaction temperature, the reaction time, and the like. However, generally, an endoprotease is preferably added in a proportion of 2,000 to 11,000 active units per g protein in the milk protein.

When the conditions for hydrolysis by the proteolytic enzyme are suitably adjusted, the milk protein hydrolysate having a specific average molecular weight range according to the invention can be obtained.

Before hydrolysis by the proteolytic enzyme, the pH of the raw material milk protein solution may be adjusted to the optimal pH for the used enzyme using edible salts, such as potassium carbonate and sodium hydroxide. The pH of the raw material milk protein solution is adjusted preferably to 5 to 10, and more preferably to 7 to 10.

The reaction of the proteolytic enzyme is desirably performed at a temperature within the optimum temperature range for the used enzyme, preferably at 30 to 60°C, and more preferably at 40 to 60°C.

The holding time of the proteolytic enzyme reaction may be suitably adjusted to make the specific non-protein nitrogen ratio, and may be 0.5 to 24 hours, for example, preferably 1 to 15 hours, and more preferably 3 to 10 hours.

Hydrolysis by the proteolytic enzyme may be completed by deactivating the enzyme by heating. For example, it is preferable that in the case where the enzyme is deactivated at 100°C or more (preferably 110 to 130°C), heating is performed for 1 to 3 seconds, while in the case where the enzyme is deactivated at less than 100°C to 60°C or more, heating is performed for 3 to 40 minutes.

After the completion of hydrolysis, it is preferable that, as necessary, the pH of the decomposition solution is made preferably 6 to 8, more preferably 7.0±0.5, and still more preferably 7.0±0.3.

Incidentally, in the production of the milk protein hydrolysate according to the invention, in the case where a solution having an unadjusted calcium concentration is hydrolyzed, the resulting decomposition solution may be subjected to the above desalting treatment to adjust the calcium concentration. Next, heating is performed in the usual manner to deactivate the enzyme. As the reaction heating temperature and the reaction holding time, conditions that can sufficiently cause deactivation can be suitably set considering the thermal stability of the used enzyme. After deactivation by heating, the reaction mixture can be cooled in the usual manner and directly used, or can also be concentrated as necessary to obtain a liquid concentrate. Furthermore, the liquid concentrate can also be dried to obtain a powder product.

In addition, when the aqueous milk protein solution is hydrolyzed by an acid treatment or an alkali treatment, the treatment may be performed by adjusting the pH of the aqueous milk protein solution. In the case of the treatment by pH adjustment, the pH of the aqueous milk protein solution is preferably pH 5 or less or pH 9 or more, and more preferably pH 4 or less or pH 10 or more. Such a pH-treated aqueous solution is allowed to stand or stirred at room temperature for several minutes or longer, preferably 5 minutes to 1 hours, whereby an acid-treated or alkali-treated hydrolysate can be obtained. Here, the "room temperature" is about 4 to 40°C, and preferably 10 to 30°C.

In addition, the aqueous milk protein solution may also be hydrolyzed by a heat treatment. Such an aqueous milk protein solution may have an unadjusted pH, or may also be subjected to pH adjustment (specifically, acidic (pH 5 or less), neutral (pH 6 to 8), or alkaline (pH 8 or more)). The heat treatment may be performed at about 4 to 100°C under conditions as in the above acid/alkali treatment.

The obtained milk protein hydrolysate can be used in an unpurified form to exert its efficacy, but may also be further suitably subjected to known separation/purification. For example, the obtained milk protein hydrolysate can be subjected to molecular weight fractionation, thereby giving a milk protein hydrolysate satisfying the ranges of average molecular weight and free amino acid percentage according to the invention.

For molecular weight fractionation, methods such as ultrafiltration and gel filtration are applicable, for example. As a result, the removal efficiency of peptides of undesirable molecular weight and free amino acids can be enhanced.

In the case of ultrafiltration, a desired ultrafiltration membrane may be used, while in the case of gel filtration, a gel filtering agent used for desired size exclusion chromatography may be used.

Furthermore, for the purpose of desalting, impurity removal, or purity enhancement, a known separation/purification method (e.g., ion exchange resin, etc.) may also be used.

In the invention, it is preferable that the milk protein hydrolysate contains at least a tripeptide having only the amino acid sequence Met-Lys-Pro or a peptide including the sequence (collectively referred to as "MKP peptide") . Here, the MKP peptide may be contained in the form of a salt thereof. Incidentally, Met, Lys, and Pro represent a methionine residue, a lysine residue, and a proline residue, respectively.

In the invention, the MKP peptide may be obtained by the decomposition treatment of a protein from soybeans, eggs, wheat, barley, rice, potatoes, sweet potatoes, green peas, corn, livestock meat, fish meat, fishery products, or the like. It is also possible that the MKP peptide is further isolated/purified from such a decomposition treatment product, or the MKP peptide is obtained by chemical synthesis or biosynthesis.

Incidentally, as used herein, "milk protein hydrolysate" as an active ingredient is the concept that also includes MKP peptides obtained by the decomposition treatment of a protein of non-milk origin and MKP peptides obtained by chemical synthesis or biosynthesis as described above.

Incidentally, in the invention, the peptide including the amino acid sequence Met-Lys-Pro refers to a peptide having a peptide residue and one or more arbitrary amino acids added to the N-terminal or C-terminal of the sequence Met-Lys-Pro. More specifically, it is preferable that the peptide has added thereto 1 to 10 residues, preferably 1 to 5 residues, and still more preferably 1 to 3 residues, and its sequence has a promoting action on energy expenditure. Preferred examples include, but are not limited to, the sequences set forth in SEQ ID NO: 1 to SEQ ID NO: 4.

In the invention, the content of the MKP peptide in the milk protein hydrolysate is not particularly limited. However, the lower limit thereof is, in terms of exerting the efficacy of the invention more favorably, preferably 0.001 mass% or more, 0.005 mass% or more, and more preferably 0.01 mass% or more, while the upper limit thereof is, in terms of the production efficiency of the hydrolysate of the invention, preferably 1 mass% or less, more preferably 0.5 mass% or less, still more preferably 0.2 mass% or less, and yet more preferably 0.1 mass% or less. The content of the MKP peptide is more preferably 0.001 to 1 mass%, still more preferably 0.005 to 0.5 mass%, and yet more preferably 0.01 to 0.1 mass%.

Incidentally, in the invention, the MKP peptide content can be measured by the following method.

(a) A sample powder is diluted and dissolved in a 0.2% aqueous formic acid solution to 1.0 mg/mL, ultrasonically crushed for 10 minutes, and then filtered through a PVDF filter having a diameter of 0.22 $\mu$m (manufactured by Millipore) to prepare a powder solution, followed by LC/MS analysis under the following measurement conditions. Meanwhile, several solutions of a chemically synthesized standard peptide for the MKP peptide (manufactured by Peptide Institute, Inc.) having different concentrations are prepared and subjected to LC/MS analysis under the following measurement conditions, and a calibration curve is prepared.

Among the peaks in the analysis of the powder solution, one whose molecular weight and retention time are identical to those of the standard peptide is identified as having the same sequence as the standard peptide. From the comparison between the peak area of the standard peptide and the peak area of the sample powder, the MKP peptide content in the powder solution is determined.

(b) MKP Peptide Content (mg/g casein hydrolysate)

```
MKP content (mg/g casein hydrolysate) = [measured MKP peptide
value (mg) in the obtained casein hydrolysate]/[mass of the obtained
casein hydrolysate (g)]
```

The [measured MKP peptide value (mg) in the obtained casein hydrolysate] is the MKP peptide value in a sample measured by the following "LC/MS".

(c) LC/MS Apparatus Used

Mass spectrometer: TSQ Quantum Discovery MAX (manufactured by Thermo Fisher Scientific Inc.).

High-performance liquid chromatograph: Prominence (manufactured by Shimadzu Corporation), Column: XBridge BEH300 C18 $\phi$2.1 mm × 250 mm, 3.5 $\mu$m (manufactured by Waters Corporation).

(d) LC/MS Measurement Conditions

Mobile phase A: 0.2 wt% Formic acid-aqueous solution
Mobile phase B: 0.2 wt% Formic acid-acetonitrile solution
Time program: 2% B (0.0 minute) -25% B (5.0 minutes) -65% B (5.1 minutes)-65% B (10 minutes)-85% B (10.1 minutes)-85% B (13.0%)-2% B (13.1 minutes)-STOP (30.0 minutes).
Sample injection volume: 10 $\mu$L, column temperature: 40°C, liquid flow rate: 200 $\mu$L/min
Analysis mode: SRM measurement.
Product Mass: m/z = 260.10 (Parent m/z = 375.21)

The milk protein hydrolysate according to the invention has a promoting action on energy expenditure.

With respect to energy expenditure, energy expenditure can be determined by indirect calorimetry. Energy expenditure can be calculated in accordance with the method of Tadaishi et al., PLoS One. 20116 (12) e28290. (literature title, etc.) 2011, for example, using the following formula:

$$\text{Energy expenditure (cal/min)} = 3.91\ VO_2\ (ml/min) + 1.10\ VCO_2\ (ml/min)$$

In the invention, energy expenditure is not particularly limited, but is considered to include energy expenditure by basal metabolism, energy expenditure by diet-induced thermogenesis resulting from meals, energy expenditure by non-exercise activity thermogenesis resulting from routine physical activities, and thermogenesis resulting from exercise.

Incidentally, as used herein, exercise means activities performed intentionally in a planned manner for health, as a workout, or for fun, such as sports, walking, and jogging, while physical activities are physical activities in daily life excluding exercise, such as labor, housework, commuting to work/school, and hobbies.

In addition, in the invention, the promotion of energy expenditure includes that energy expenditure is strengthened from the ordinary level to increase energy expenditure, the energy expenditure level that has decreased due to various causes is recovered to increase energy expenditure to the ordinary level or higher, and/or energy expenditure that increases after exercise is further promoted.

Therefore, the milk protein hydrolysate according to the invention can be used for the purpose of promoting energy expenditure, or for the purpose of treating, alleviating, and/or preventing impaired energy expenditure. Furthermore, effectiveness can be expected in: improving energy expenditure; controlling various conditions or diseases associated with impaired energy expenditure, including immune activity enhancement, physical strength enhancement, blood flow improvement, fatigue alleviation, and obesity prevention and/or alleviation; dieting; preventing and/or alleviating metabolic syndrome; and preventing and/or alleviating lifestyle-related diseases such as diabetes.

Furthermore, because the milk protein hydrolysate according to the invention can further promote energy expenditure that increases after exercise, when it is administered during exercise and/or after exercise, energy expenditure that increases after exercise can be further increased. Accordingly, the milk protein hydrolysate according to the invention can be expected to enhance the sweating of the whole body, enhance the hyperthermic effect of the body, and promote metabolism, for example, and can be expected to be effective in obesity prevention, dieting, and metabolic syndrome prevention or alleviation.

Generally, energy expenditure is mostly lost as heat (edited by Yoshimitsu Inoue, Norihiko Kondo, *"Taion II-Taionchosetsu Shisutemu To Sono Tekiou* (Body Temperature II-Thermoregulation System and Its Adaptation)", NAP Limited). Accordingly, the milk protein hydrolysate according to the invention offers an effect of raising a body temperature, particularly raising a core temperature. Therefore, the composition for promoting energy expenditure of the invention can be used for raising a body temperature.

The milk protein hydrolysate according to the invention has a body temperature raising action, and thus is expected to return a living body temperature to a normal condition or further enhance the same, whereby a drop in body temperature is prevented or alleviated, the peripheral blood flow is improved, sweating is accelerated, or a somesthesis action is caused. In addition, it can also be used for treating, alleviating, or preventing diseases resulting from a prolonged hypothermic state, such as low blood pressure, impaired visceral function (constipation, diarrhea), immunological deterioration, poor blood circulation (coldness, stiff shoulder, lower back pain, joint pain), sleep disorder, and decreased metabolism (obesity), for example.

Incidentally, preferably, the intended use of the milk protein hydrolysate according to the invention excludes a mode in which it is used for the purpose of reducing blood cholesterol or blood neutral fat or for the purpose of improving lipid metabolism.

When the energy expenditure promoting agent/composition of the invention is applied for the disease or condition described above, the application subject can be a human or a non-human animal (particularly a mammal). In addition, the application may be therapeutic use or may also be non-therapeutic use. Incidentally, "non-therapeutic" is a concept that does not include a medical practice, that is, a practice of therapeutically treating a human body.

That is, another mode of the invention is a method for promoting energy expenditure, including a step of administering the energy expenditure promoting agent/composition of the invention to a subject. Furthermore, it may also be a method for promoting energy expenditure to thereby treat, alleviate, and/or prevent the disease or condition described above, or may also be a method for raising the body temperature of a subject.

As used herein, "alleviation" means to change the disease, symptom, or condition for the better, to prevent or delay the deterioration of the disease, symptom, or condition, or to reverse, prevent, or delay the progress of the disease or symptom.

In addition, as used herein, "prevention" means to prevent or delay the onset of the disease or symptom in the application subject, or to reduce the risk of onset of the disease or symptom in the application subject.

The invention can also be regarded as use of a milk protein hydrolysate in the production of a composition for preventing and/or alleviating a disease caused by impaired energy expenditure.

The invention can also be regarded as a milk protein hydrolysate for use in preventing and/or alleviating a disease caused by impaired energy expenditure.

The invention can also be regarded as use of a milk protein hydrolysate in the prevention and/or alleviation of a disease caused by impaired energy expenditure.

The invention can also be regarded as a method for preventing and/or alleviating a disease caused by decreased energy expenditure, including administering a milk protein hydrolysate to a subject in need of the prevention and/or alleviation of a disease caused by impaired energy expenditure.

The invention can also be regarded as use of a peptide having only Met-Lys-Pro or a peptide including this amino acid sequence in the production of a composition for preventing and/or alleviating a disease caused by impaired energy expenditure.

The invention can also be regarded as a peptide having only Met-Lys-Pro or a peptide including this amino acid sequence, for use in preventing and/or alleviating a disease caused by impaired energy expenditure.

The invention can also be regarded as use of a peptide having only Met-Lys-Pro or a peptide including this amino acid sequence in the prevention and/or alleviation of a disease caused by impaired energy expenditure.

The invention can also be regarded as a method for preventing and/or alleviating a disease caused by decreased energy expenditure, including administering a peptide having only Met-Lys-Pro or a peptide including this amino acid sequence to a subject in need of the prevention and/or alleviation of a disease caused by impaired energy expenditure.

The energy expenditure promoting agent or composition of the invention can be used after blended, as an active ingredient therefor, with a pharmaceutical, a quasi drug, an external preparation for skin, a cosmetic product, a food, or the like for humans or animals for energy expenditure promotion and/or disorders, diseases, and symptoms associated with impaired energy expenditure, or for energy expenditure promotion and/or preventing, alleviating, and/or treating impaired energy expenditure, for example, as described above. In addition, the milk protein hydrolysate described herein can be used for the production of these various preparations.

The content of the milk protein hydrolysate in the composition for promoting energy expenditure of the invention can be set at 0.001 to 100 mass% relative to the final composition.

In addition, the content of the MKP peptide in the composition for promoting energy expenditure of the invention can be set at 0.00001 to 1 mass% relative to the final composition.

Incidentally, as used herein, a final composition means the composition at the time of administration to the subject or at the time of ingestion by the subject.

In the invention, the amount of milk protein hydrolysate used relative to the subject is preferably 0.0001 to 1 g/kg body weight/day, more preferably 0.0005 to 0.5 g/kg body weight/day, and still more preferably 0.0001 to 0.1 g/kg body weight/day.

<Pharmaceutical>

In a preferred mode, the composition for promoting energy expenditure of the invention is a pharmaceutical. In that case, the pharmaceutical may be in an oral or parenteral dosage form, for example.

The pharmaceutical composition according to one mode of the composition for promoting energy expenditure of the invention may be for oral administration or parenteral administration, but is preferably for oral administration. Examples of parenteral administration include intravenous injection, rectal administration, and inhalation. Such a pharmaceutical can be suitably formulated into a desired dosage form according to the administration method. For example, in the case of oral administration, the pharmaceutical can be formulated into a solid preparation such as a powder, granules, tablets, or capsules; a liquid preparation such as a solution, a syrup, a suspension, or an emulsion; or the like. In addition, in the case of parenteral administration, the pharmaceutical can be formulated into a suppository, an ointment, or the like.

For formulation, in addition to the milk protein hydrolysate described herein, components usually used for formulation, such as diluents, pH adjusters, colorants, and correctives, can be used. In addition, together therewith, it also possible to use a drug known or later discovered to have the above energy expenditure promoting action, a drug for alleviating abnormal lipid metabolism, a drug for treating hyperlipemia, or the like.

In addition, formulation can be suitably performed by a known method according to the dosage form. For formulation, a formulation carrier may be suitably blended and formulated.

Examples of diluents include sugar derivatives such as lactose, sucrose, glucose, mannite, and sorbitol; starch derivatives such as corn starch, potato starch, α-starch, dextrin, and carboxymethyl starch; cellulose derivatives such as crystalline cellulose, hydroxypropyl cellulose, hydroxypropylmethyl cellulose, carboxymethyl cellulose, and carboxymethyl cellulose calcium; gum arabic; dextran; pullulan; silicate derivatives such as light anhydrous silicic acid, synthetic aluminum silicate, and magnesium aluminometasilicate; phosphate derivatives such as calcium phosphate; carbonate derivatives such as calcium carbonate; and sulfate derivatives such as calcium sulfate.

Examples of binders include, in addition to the above diluents, gelatin; polyvinylpyrrolidone; and macrogol.

Examples of disintegrators include, in addition to the above diluents, chemically modified starch or cellulose derivatives, such as cross carmellose sodium, sodium carboxy methyl starch, and crosslinked polyvinylpyrrolidone.

Examples of lubricants include talc; stearic acid; metal stearates such as calcium stearate and magnesium stearate; colloidal silica; waxes such as beeswax and spermaceti; boric acid; glycol; carboxylic acids such as fumaric acid and adipic acid; sodium carboxylates such as sodium benzoate; sulfates such as sodium sulfate; leucine; lauryl sulfates such as sodium lauryl sulfate and magnesium lauryl sulfate; silicic acids such as silicic anhydride and silicic acid hydrate; and starch derivatives.

Examples of stabilizers include p-oxybenzoate esters such as methylparaben and propylparaben; alcohols such as chlorobutanol, benzyl alcohol, and phenyl ethyl alcohol; benzalkonium chloride; acetic anhydride; and sorbic acid.

Examples of correctives include sweeteners, acidulants, and flavoring agents.

Incidentally, examples of carriers used in the case of a solution for oral administration include solvents such as water.

The timing of ingesting the pharmaceutical composition of the invention is not particularly limited, but it is preferably ingested or administered within 2 hours before a meal to 2 hours after a meal, for example, and more preferably within 1 hour before a meal to 1 hour after a meal. In addition, the condition before and before ingestion may be exercise or rest. Further, the energy expenditure promoting agent of the invention is preferably ingested or administered within 1 hour before exercise to 1 hour after exercise, and particularly preferably ingested or administered from immediately before the start of exercise to immediately after the completion of exercise.

<Food or Beverage Composition>

In the case where the energy expenditure promoting agent or composition of the invention is blended with a food, such a food can be offered/sold as a food or beverage with a label stating the intended use described above, that is, the promotion of energy expenditure and/or the prevention, alleviation, or treatment of various diseases and the like caused by impaired energy expenditure . In addition, the milk protein hydrolysate described herein can be used for the production of these foods and the like.

Such "labeling" includes all acts for making consumers aware of the above intended use. As long as the expression allows consumers to assume or infer the intended use, all such acts fall within the "labeling" of the invention regardless of the purpose of the label, the content of the label, the object/medium to be labeled, and the like.

In addition, it is preferable that "labeling" is performed with an expression that allows consumers to directly recognize the intended use. Specific examples thereof are the following acts: a food- or beverage-related product stating the intended use thereon or on the package thereof is transferred, delivered, displayed for transfer or delivery, or imported; the intended use is described in an advertisement, price list, or transaction document related to the product and displayed or distributed, or the intended use is described in information having such contents and provided through an electromagnetic means (Internet, etc.); and the like.

Meanwhile, with respect to the content of the label, it is preferable that the label has been approved by the government or the like (e.g., a label that has been approved based on systems established by the government and attached in a mode in accordance with the approval, etc.) . In addition, it is preferable that such a content of the label is attached to packages, containers, catalogs, pamphlets, POPs and like advertising materials at the sales location, or other documents.

In addition, "labeling" also includes labeling for health foods, functional foods, enteral foods, special purpose foods, foods with health claims, foods for specified health uses, foods with nutrient function claims, foods with function claims, quasi drugs, and the like. Among them, in particular, labels approved by the Consumer Affairs Agency, such as labels approved based on a system concerning foods for specified health uses, foods with nutrient function claims, or foods with function claims or on a similar system, can be mentioned. Specific examples thereof include labels for foods for specified health uses, labels for qualified foods for specified health uses, labels to inform that the body structure or function may be affected, labels to show disease risk reduction, and labels to show evidence-based functionality. More specifically, typical examples are labels for foods for specified health uses (particularly health use claims) under Cabinet Office Ordinance on Permission for Special Use Claims, etc., prescribed in Health Promotion Act (August 31, 2009; Cabinet Office Order No. 57) and similar labels.

As such labels, for example, to target those who are doing exercise or on diet, it is possible to attach a label stating "those who want to enhance energy expenditure", "those who want to enhance metabolism", "those who want to reduce body fat", and the like.

Alternatively, to target elderly persons and/or those with decreased immune activity and those with decreased basal metabolic capability, it is possible to attach a label stating "those who worry about a decrease in basal metabolism", "those who want to warm the body", "those with excessive cold sensitivity", "those who are feeling fatigue", and the like.

In addition, as one mode of the milk protein hydrolysate described herein and various preparations containing the same as an active ingredient described above, the milk protein hydrolysate described herein can be contained in a food or beverage as an active ingredient and processed into a food having the above energy expenditure promoting action and the like.

Such a food may be in any form, such as a liquid, a paste, a solid, a powder, or the like. Examples thereof include tablet confectioneries, liquid foods, and animal feeds (including feeds for pets), as well as flour products, instant foods, processed agricultural products, processed fishery products, processed livestock products, milk/dairy products, oils and fats, basic seasonings, composite seasonings/foods, frozen foods, confectionaries, beverages, and other commercially available foods.

Examples of the flour products include bread, macaroni, spaghetti, noodles, cake mixes, seasoned flour for fried chicken, and bread crumbs.

Examples of the instant foods include instant noodles, instant noodles in cups, retort-packaged/cooked foods, canned cooked foods, microwave foods, instant soups/stews, instant miso soup/clear soups, canned soups, freeze-dried foods, and other instant foods.

Examples of the processed agricultural products include canned agricultural products, canned fruits, jams/marmalades, pickles, simmered legumes, dried agricultural products, and cereals (processed grain products).

Examples of the processed fishery products include canned fishery products, fish meat hams/sausages, pasted fishery products, fishery delicacies, and *tsukudani* (preservable food boiled down in sweetened soy sauce).

Examples of the processed livestock products include canned/pasted livestock products and livestock meat hams/sausages.

Examples of the milk/dairy products include processed milk, milk beverages, yogurts, lactic acid bacteria beverages, cheeses, ice creams, modified milk powders, creams, and other dairy products.

Examples of the oils and fats include butter, margarine, and vegetable oils.

Examples of the basic seasonings include soy sauce, miso, sauces, processed tomato seasonings, *mirin* (sweet cooking sake), and vinegars.

Examples of the composite seasonings/foods include cooking mixes, curry bases, sauces, dressings, noodle seasonings, spices, and other composite seasonings . For example, examples of the frozen foods include frozen raw foods, frozen semi-cooked foods, and frozen cooked foods.

Examples of the confectionaries include caramels, candies, chewing gums, chocolates, cookies, biscuits, cakes, pies, snacks, crackers, Japanese sweets, rice confectioneries, bean confectioneries, dessert confectioneries, and other confectioneries.

Examples of the beverages include carbonated beverages, natural fruit juices, fruit juice beverages, fruit juice-containing soft beverages, fruit pulp beverages, granule-containing fruit beverages, vegetable-based beverages, soy milk, soy milk beverages, coffee beverages, tea beverages, powdered beverages, concentrated beverages, sports beverages, nutritional beverages, alcoholic beverages, and other fancy drinks.

Examples of commercially available foods other than those mentioned above include baby foods, *furikake* (seasoning powder for rice), and *ochazuke-nori* (seasoning powder for rice soup).

In the energy expenditure promoting agent according to the invention and also in the pharmaceutical and food or beverage composition containing the same, the content of the milk protein hydrolysate described herein is preferably at least 0.001 mass% relative to the final agent or composition.

The intake or dose of the milk protein hydrolysate described herein varies depending on the age, symptoms, and the like, but is usually 0.001 g to 0.5 g/kg body weight/day, preferably 0.006 g to 0.334 g/kg, still more preferably 0.015 g to 0.167 g/kg body weight, and particularly preferably 0.03 g to 0.1 g/kg body weight, and it may be administered in one to three doses per day.

The intake or dose of the MKP peptide described herein varies depending on the age, symptoms, and the like, but is usually 0.4 $\mu$g to 200 $\mu$g/kg body weight/day, preferably 2.4 $\mu$g to 130 $\mu$g/kg, still more preferably 6 $\mu$g to 66.8 $\mu$g/kg body weight, and particularly preferably 12 $\mu$g to 40 $\mu$g/kg body weight, and it may be administered in one to three doses per day.

Incidentally, because the milk protein hydrolysate is a protein, from the ratio between the daily protein intake and the milk protein hydrolysate intake, the dosage for administration to a human can be estimated.

When the body weight of a mouse is 22 g, the dose of the milk protein hydrolysate to a mouse (protein equivalent unit) is calculated to be about 11 to 22 mg/individual. When the feeding amount is 3.5 g/day, and the amount of protein in the feed is 20.7%, the calculated dosage is equivalent to about 1.5 to 3.0 mass% of the protein ingested from the feed. This can be applied to a human as follows: when the daily protein intake is 60 g for an adult male and 50 g for an adult female, the dosage is calculated to be about 0.9 to 1.8 g for an adult male and about 0.75 to 1.5 g for an adult female.

Examples

Hereinafter, the invention will be described in further detail using examples. However, the invention is not limited to these examples.

<1> Preparation of Milk Protein Hydrolysate

(1) Preparation of Casein Hydrolysate

To 100 g of a commercially available casein (manufactured by New Zealand Dairy Board) was added 900 g of water, and well dispersed. Sodium hydroxide was added to adjust the pH of the solution to 7.0, and the casein was completely dissolved, thereby preparing an aqueous casein solution having a concentration of about 10 mass%. The aqueous casein solution was heat-sterilized at 85°C for 10 minutes, the temperature was adjusted to 50°C, and sodium hydroxide was added to adjust the pH to 9.5. Subsequently, 100,800 active units of Bioprase sp-20 (manufactured by Nagase Biochemicals, Ltd.) (1,200 active units per g protein), 168, 000 active units of Protease N (manufactured by Amano Enzyme Inc.) (2,000 active units per g protein), and 588,000 active units of PTN6.0S (manufactured by Novozymes Japan Ltd.) (7,000 active units per g protein) were added to initiate a hydrolysis reaction. At the time when the casein decomposition rate reached 24.1%, the mixture was heated at 80°C for 6 minutes to deactivate the enzymes and stop the enzyme reaction, followed by cooling to 10°C. The hydrolysis solution was ultrafiltered through an ultrafiltration membrane having a molecular weight cutoff of 3,000 (manufactured by Asahi Kasei Corporation), concentrated, and then lyophilized to give a lyophilized product.
The molecular weight and the free amino acid of the obtained hydrolysate were measured by the methods described above. As a result, a casein hydrolysate in which the average molecular weight was 360 Da, the percentage of free amino acids was 10 mass% or less, and peptides having a molecular weight of 200 to 500 were contained in an amount of 53 mass% was obtained.

(2) Preparation of Whey Protein Hydrolysate

2.6 kg of a commercially available cow milk whey protein concentrate (manufactured by Milei GmbH, protein equivalent: 2 kg) was dissolved in 18 kg of deionized water, and, using a 10% aqueous sodium hydroxide solution, the pH was adjusted to 7.2, thereby preparing 20.6 kg of an aqueous whey protein solution having a protein concentration of about 10%. The aqueous whey protein solution was heat-sterilized at 80°C for 6 minutes using a plate-type heat exchanger, the liquid temperature was adjusted to 53°C, and the pH was adjusted to 9.0 using a 10% aqueous potassium hydroxide solution. PTN 6.0S (manufactured by Novozymes) which is an endoprotease, Protease N Amano (manufactured by Amano Enzyme Inc.) , and Alcalase 2.4 L (manufactured by Amano Enzyme Inc.) were added in proportions of 5,000 active units, 2,000 active units, and 500 active units per g protein, respectively, to initiate a protein hydrolysis reaction. After 10 hours, at the time when the decomposition rate reached 20%, the mixture was heated at 120°C for 3 seconds to deactivate the enzymes and stop the enzyme reaction, followed by cooling to 10°C.
The hydrolysis solution was subjected to membrane fractionation through a microfiltration membrane having a pore size of 0.45 μm (manufactured by Millipore) to give about 18 kg of an aqueous protein hydrolysate solution as permeate. The liquid temperature of the aqueous protein hydrolysate solution was adjusted to 55°C, and Protease A Amano (manufactured by Amano Enzyme Inc.), which is an exoprotease, was added in a proportion of 500 active units per g protein equivalent to initiate a second protein hydrolysis reaction. After 10 hours, the decomposition rate was increased by 7%, and, at the time when the final decomposition rate reached 27%, the mixture was heated using a plate-type heat exchanger at 120°C for 3 seconds to deactivate the enzyme and stop the enzyme reaction, followed by cooling to 10°C.
The molecular weight and the free amino acids of the obtained whey hydrolysate were measured by the methods described above. As a result, the average molecular weight was 360 Da, the percentage of free amino acids was 12 mass% or less, and the peptides having a molecular weight of 200 to 500 were contained in an amount of 45 mass%.

(3) Preparation of MKP Peptide 1

To 100 g of a commercially available casein (manufactured by New Zealand Dairy Board) was added 900 g of water, and well dispersed . Sodium hydroxide was added to adjust the pH of the solution to 7.0, and the casein was completely dissolved, thereby preparing an aqueous casein solution having a concentration of about 10%. The aqueous casein solution was heat-sterilized at 85°C for 10 minutes, the temperature was adjusted to 50°C, and sodium hydroxide was added to adjust the pH to 9.5. Subsequently, 100,800 active units of Bioprase sp-20 (manufactured by Nagase Biochemicals, Ltd.) (1,200 active units per g protein), 168,000 active units of Protease N (manufactured by Amano Enzyme Inc.) (2,000 active units per g protein), and 588,000 active units of PTN6.0S (manufactured by Novozymes Japan Ltd.) (7,000 active units per g protein) were added to initiate a hydrolysis reaction. At the time when the casein decomposition rate reached 24.1%, the mixture was heated at 80°C for 6 minutes to deactivate the enzymes and stop the enzyme reaction, followed by cooling to 10°C. The hydrolysis solution was ultrafiltered through an ultrafiltration membrane having a molecular weight cutoff of 3,000 (manufactured by Asahi Kasei Corporation), concentrated, and then lyophilized to give 85g of a lyophilized product.

The casein hydrolysate obtained above was subjected to separation by reversed-phase HPLC. The HPLC conditions were as shown in the following Conditions 1:

HPLC Conditions 1:

Column: Capsule pack C18 (UG120, 5 $\mu$m) 20 mm I.D. $\times$ 250 mm (Shiseido)
Detection: UV 215 nm
Flow rate: 16 ml/min
Eluent A: 1% Aqueous acetonitrile solution containing 0.05% TFA
Eluent B: 25% Aqueous acetonitrile solution containing 0.05% TFA

The hydrolysate was separated under linear gradient conditions to reach 100% Eluent B in 40 minutes from 100% Eluent A.

In order to purify peptides contained in the fraction eluted at a retention time of 22 minutes, further separation by HPLC was performed under the following Conditions 2:

HPLC Conditions 2:

Column: Capsule pack C18 (UG300, 5 $\mu$m) 2.0 mm I.D. $\times$ 250 mm (Shiseido)
Detection: UV 215 nm
Flow rate: 0.2 ml/min
Eluent A: 1% Aqueous acetonitrile solution containing 0.05% TFA
Eluent B: 10% Aqueous acetonitrile solution containing 0.05% TFA

Under linear gradient conditions to reach 100% Eluent B in 15 minutes from 100% Eluent A, peptides contained in the peak at a retention time of 13 minutes were lyophilized.

Incidentally, it was confirmed that the lyophilized product contained a tripeptide having the structure H-Met-Lys-Pro-OH (MKP peptide) as described in PTL 4.

The MKP peptide content in 85 g of the lyophilized product was 43.5 mg.

(4) Preparation of MKP Peptide 2

Using a peptide synthesizer (Model 433A, Applied Biosystems), and using Fmoc-L-Met (Applied Biosystems), Fmoc-Lys (Boc) (Applied Biosystems), and Fmoc-Pro-TrtA-PEG Resin (Watanabe Chemical Industries, Ltd.) as raw materials, a tripeptide Met-Lys-Pro was synthesized by a solid-phase synthesis method. The operation was performed according to the manual of Applied Biosystems, followed by deprotection. The peptide was purified under the HPLC Conditions 1 described above. The purified product was subjected to mass spectrometry and confirmed to be a MKP peptide.

<2> Administration of Milk Protein Hydrolysate

The energy expenditure promoting action of the milk protein hydrolysate was examined through the following examples.

(1) Administration after Exercise

Male C57BL/6J mice were fasted for 16 hours and then made to run on a treadmill at 15 m/min for 60 minutes. Subsequently, the mice were divided into three groups (n = 8 in each group), and a carbohydrate and a casein hydrolysate (Glu + Pep, 1.5 mg + 0.5 mg/g body weight), a carbohydrate and an amino acid mixture of the same composition as a casein hydrolysate (Glu + AA, 1.5 mg + 0.5 mg/g body weight), and a carbohydrate alone (Glu, 1.5 mg/g body weight) were orally administered to the respective groups. The amino acid mixture of the same composition as a casein hydrolysate was prepared by mixing 20 kinds of crystalline amino acids according to the amino acid composition of the casein hydrolysate having completely decomposed amino acids. Incidentally, glucose was used as the carbohydrate, and, as the casein hydrolysate, the one prepared in <1> above was used. Expired gas was continuously collected from 30 minutes to 120 minutes after administration, and, from the oxygen concentration and carbon dioxide concentration in the expired air, energy expenditure was determined by indirect calorimetry. Incidentally, for the calculation of energy expenditure, in accordance with the method of Tadaishi et al. , PLoS One. 20116 (12), e28290., the following formula was used.

$$\text{Energy expenditure (cal/min)} = 3.91 \ VO_2 \ (ml/min) + 1.10 \ VCO_2 \ (ml/min).$$

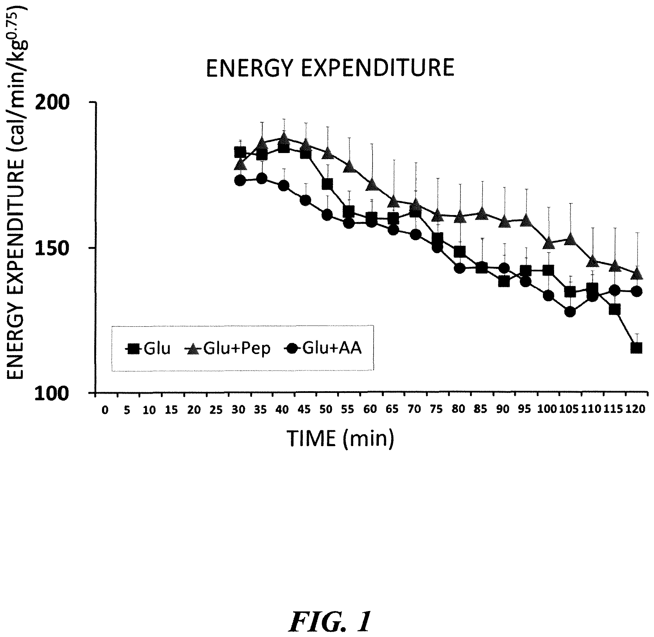

In addition, for the statistical processing, the two-way analysis of variance and Tukey's HSD Test were used. The results of the energy expenditure measurement are shown in Fig. 1. As compared with the group administered with a carbohydrate alone and the group administered with a carbohydrate and an amino acid mixture, in the group administered with a carbohydrate and a casein hydrolysate, the level of energy expenditure was significantly higher ($p < 0.01$). From these results, it can be seen that the ingestion of a casein hydrolysate after exercise is more effective in increasing energy expenditure as compared with the ingestion of amino acids of the same composition.

(2) Administration at Rest 1

Male C57BL/6J mice were fasted for 1 hour and then divided into four groups (n = 7 in each group), and a casein hydrolysate (Pep), a casein (Cas), an amino acid mixture of the same composition as a casein hydrolysate (AA), and water (Water) were orally administered in an amount of 1.0 mg/g body weight to the respective groups. Incidentally, as the casein hydrolysate, the one prepared in <1> above was used, and, as the casein, the raw material before hydrolysis in <1> was used. Energy expenditure from 5 minutes to 120 minutes after administration was determined in the same manner as in (1) above. In addition, for the statistical processing, as in (1), the two-way analysis of variance and Tukey's HSD Test were used.

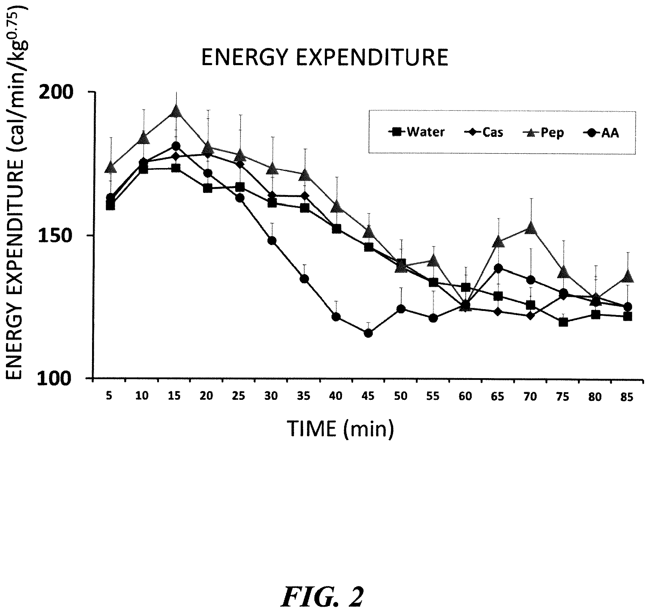

The results of the energy expenditure measurement are shown in Fig. 2. As compared with the groups administered with water or an amino acid mixture, the group that ingested a casein hydrolysate resulted in a significantly higher level of energy expenditure ($p < 0.01$). In addition, also in comparison with the group administered with a casein, in the group that ingested a casein hydrolysate, the level of energy expenditure was higher ($p < 0.05$). From these results, it can be seen that as compared with the ingestion of a casein in the form of a protein or amino acids, when a casein is ingested after hydrolysis, that is, in the form of a casein hydrolysate, energy expenditure is promoted more efficiently.

(3) Administration at Rest 2

Male C57BL/6J mice as laboratory animals were divided into the following four groups: rest group, casein hydrolysate administration group, MKP peptide administration group, and whey protein hydrolysate administration group. The mice were fasted for 1 hour. Subsequently, a casein hydrolysate and a whey protein hydrolysate each in an amount of 1.0 mg/g body weight and a MKP peptide in an amount of 0.4 μg/g body weight were orally administered, and energy expenditure was measured. The number of mice used in the experiment was n = 12 in each group, and a randomized crossover test was employed.

Incidentally, as the casein hydrolysate and the whey protein hydrolysate, those prepared in <1>, (1) to (2), above were used, and, as the MKP peptide, a synthetic peptide was purchased from Peptide Institute and used. Energy expenditure for 60 minutes after administration was determined.

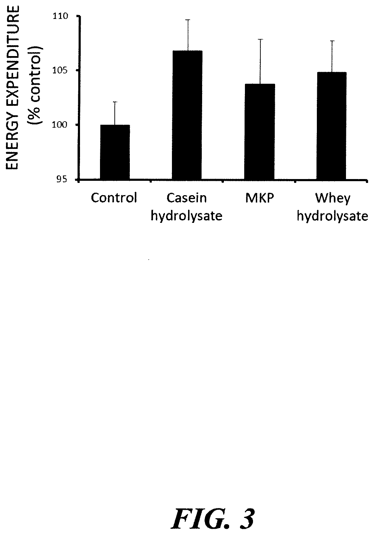

The average energy expenditure for 60 minutes after administration is shown in Fig. 3. As compared with the rest group, in all the casein hydrolysate administration group, MKP peptide administration group, and whey protein hydrolysate administration group, the level of energy expenditure was higher. From these results, it can be seen that as a result of ingesting a hydrolysate of a casein or whey protein, which constitutes a milk protein, or a MKP peptide, energy expenditure increases.

<Production Example 1>

The casein hydrolysate produced in Example 1, sucralose (manufactured by San-Ei Gen F. F. I., Inc.)., and a rare monosaccharide (trade name: Rare Sugar Sweet (manufactured by Matsutani Chemical Industry Co., Ltd.)) are blended with cow milk or defatted milk to give a liquid milk preparation containing (A) milk protein: 3.5mass% or more, (B) milk fat: 3.5 mass% or less, (C) carbohydrate: 5.0 mass% or more, and (D) calcium: 0.15 mass% or more.

The liquid milk preparation, lactic acid bacteria, and bifidobacteria are uniformly mixed, and the resulting mixture is fermented. As a result, a composition for energy expenditure containing a casein hydrolysate and indigestible dextrin (fermented milk) is obtained. The fermented milk contains (E) the casein hydrolysate of Example 1: 0.3 to 0.8 mass%, (F) indigestible dextrin: 4 to 8 mass%, (G) sucralose: 0.05 to 0.02 mass%, (H) D-psicose and D-allose: 0.05 to 0.1 mass%, and (I) lactulose: 1 to 4 mass%.

The composition for energy expenditure of the invention is continuously ingested every day in such a manner that the casein hydrolysate intake is 0.0001 to 1 g/kg body weight/day. This can be expected to have an energy expenditure promoting effect.

<Production Example 2>

The components (powders) shown in Table 1 are mixed to give a powder composition for energy expenditure. The powder can be used as a supplement, and can also be mixed with water and served as a beverage. In addition, the powder can be placed in a capsule container or encapsulated to give a capsule for energy expenditure. The powder can be pressed to give a tablet confectionery for promoting energy expenditure. The powder can be expected to have an energy expenditure promoting effect. Incidentally, the casein hydrolysate as a raw material of the powder can be the casein hydrolysate of Example 1.

[Table 1]

| Raw Material (Powder) | Blending Proportion [mass%] | Blending Amount [mg/package] |
|---|---|---|
| Casein hydrolysate | 8.500 | 510 |
| Flavoring agent preparation | 4.500 | 270 |
| Tricalcium phosphate | 1.000 | 60 |
| Sweetener preparation | 1.000 | 60 |
| Total including other diluents | 100.000 | 6,000 |

## Claims

1. A composition for promoting energy expenditure, comprising a milk protein hydrolysate having an average molecular weight of 220 Daltons or more and 1,000 Daltons or less as an active ingredient.

2. The composition for promoting energy expenditure according to claim 1, for use in raising a body temperature.

3. The composition for promoting energy expenditure according to claim 1 or 2, for ingestion after exercise.

4. The composition for promoting energy expenditure according to any one of claims 1 to 3, wherein the milk protein is a casein.

5. The composition for promoting energy expenditure according to any one of claims 1 to 3, wherein the milk protein is a whey protein .

6. The composition for promoting energy expenditure according to any one of claims 1 to 5, wherein the milk protein hydrolysate is a hydrolysate by a proteolytic enzyme.

7. The composition for promoting energy expenditure according to any one of claims 1 to 6, wherein the milk protein hydrolysate contains at least a peptide consisting of Met-Lys-Pro or a peptide including the amino acid sequence.

8. The composition for promoting energy expenditure according to any one of claims 1 to 7, wherein the energy expenditure is attributable to a factor selected from the group consisting of basal metabolism, diet-induced thermogenesis, non-exercise activity thermogenesis, and thermogenesis due to exercise.

9. The composition according to any one of claims 1 to 8, being a pharmaceutical composition for treating and/or preventing impaired energy expenditure or for promoting energy expenditure.

10. A food or beverage composition for promoting energy expenditure, comprising a milk protein hydrolysate having an average molecular weight of 220 Daltons or more and 1,000 Daltons or less as an active ingredient.

11. The food or beverage composition according to claim 10, for use in raising a body temperature.

12. The food or beverage composition according to claim 10 or 11, for ingestion after exercise.

13. The food or beverage composition according to any one of claims 10 to 12, wherein the milk protein is a casein.

14. The food or beverage composition according to any one of claims 10 to 12, wherein the milk protein is a whey protein.

15. The food or beverage composition according to any one of claims 10 to 14, wherein the milk protein hydrolysate is a hydrolysate by a proteolytic enzyme.

16. The food or beverage composition according to any one of claims 10 to 15, wherein the milk protein hydrolysate contains at least a peptide consisting of Met-Lys-Pro or a peptide including the amino acid sequence.

17. The food or beverage composition according to any one of claims 10 to 16, wherein the energy expenditure is attributable to a factor selected from the group consisting of basal metabolism, diet-induced thermogenesis, non-exercise activity thermogenesis, and thermogenesis due to exercise.

18. A composition for promoting energy expenditure, comprising a peptide consisting of Met-Lys-Pro or a peptide including the amino acid sequence, as an active ingredient.

19. The composition for promoting energy expenditure according to claim 18, wherein the energy expenditure is attributable to a factor selected from the group consisting of basal metabolism, diet-induced thermogenesis, non-exercise activity thermogenesis, and thermogenesis due to exercise.

20. Use of a milk protein hydrolysate in the production of a composition for preventing and/or alleviating a disease caused by impaired energy expenditure.

21. A milk protein hydrolysate for use in preventing and/or alleviating a disease caused by impaired energy expenditure.

22. Use of a milk protein hydrolysate in the prevention and/or alleviation of a disease caused by impaired energy expenditure.

23. A method for preventing and/or alleviating a disease caused by decreased energy expenditure, comprising administering a milk protein hydrolysate to a subject in need of the prevention and/or alleviation of a disease caused by impaired energy expenditure.

24. Use of a peptide consisting of Met-Lys-Pro or a peptide including the amino acid sequence in the production of a composition for preventing and/or alleviating a disease caused by impaired energy expenditure.

25. A peptide consisting of Met-Lys-Pro or a peptide including the amino acid sequence, for use in preventing and/or alleviating a disease caused by impaired energy expenditure.

26. Use of a peptide consisting of Met-Lys-Pro or a peptide including the amino acid sequence in the prevention and/or alleviation of a disease caused by impaired energy expenditure.

27. A method for preventing and/or alleviating a disease caused by decreased energy expenditure, comprising administering a peptide consisting of Met-Lys-Pro or a peptide including the amino acid sequence to a subject in need of the prevention and/or alleviation of a disease caused by impaired energy expenditure.

**FIG. 1**

FIG. 2

*FIG. 3*

<table>
<tr><td colspan="2">**INTERNATIONAL SEARCH REPORT**</td><td>International application No.</td></tr>
<tr><td colspan="2"></td><td>PCT/JP2018/011811</td></tr>
</table>

**A. CLASSIFICATION OF SUBJECT MATTER**
Int. Cl. A61K38/01(2006.01)i, A23L33/19(2016.01)i, A61K38/16(2006.01)i,
A61P3/00(2006.01)i

According to International Patent Classification (IPC) or to both national classification and IPC

**B. FIELDS SEARCHED**

Minimum documentation searched (classification system followed by classification symbols)
Int. Cl. A61K38/01, A23L33/19, A61K38/16, A61P3/00

Documentation searched other than minimum documentation to the extent that such documents are included in the fields searched
Published examined utility model applications of Japan      1922-1996
Published unexamined utility model applications of Japan    1971-2018
Registered utility model specifications of Japan            1996-2018
Published registered utility model applications of Japan    1994-2018

Electronic data base consulted during the international search (name of data base and, where practicable, search terms used)
JSTPlus/JMEDPlus/JST7580 (JDreamIII),
CAplus/REGISTRY/BIOSIS/CABA/AGRICOLA/FSTA(STN)

**C. DOCUMENTS CONSIDERED TO BE RELEVANT**

| Category* | Citation of document, with indication, where appropriate, of the relevant passages | Relevant to claim No. |
|---|---|---|
| X | WO 2003/044044 A1 (MORINAGA MILK INDUSTRY CO., LTD.) 30 May 2003, claims & US 2005/0004041 A1, claims & EP 1457498 A1 | 25 |
| Y | WO 2007/142230 A1 (MORINAGA MILK INDUSTRY CO., LTD.) 13 December 2007, examples & US 2009/0012001 A1, examples & EP 2030629 A1 | 1-27 |
| Y | JP 2010-248136 A (SNOW BRAND MILK PRODUCTS CO., LTD.) 04 November 2010, claims, examples & US 2012/0071400 A1, claims, examples & WO 2010/119803 A1 & EP 2420244 A1 | 1-27 |

☒ Further documents are listed in the continuation of Box C.    ☐ See patent family annex.

| | |
|---|---|
| *   Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| 15.06.2018 | 26.06.2018 |

| Name and mailing address of the ISA/<br>    Japan Patent Office<br>    3-4-3, Kasumigaseki, Chiyoda-ku,<br>    Tokyo 100-8915, Japan | Authorized officer<br><br><br>Telephone No. |

Form PCT/ISA/210 (second sheet) (January 2015)

**INTERNATIONAL SEARCH REPORT**

| International application No. |
|---|
| PCT/JP2018/011811 |

C (Continuation).   DOCUMENTS CONSIDERED TO BE RELEVANT

| Category* | Citation of document, with indication, where appropriate, of the relevant passages | Relevant to claim No. |
|---|---|---|
| Y | BENDTSEN, Line Q. et al., Effects of hydrolysed casein, intact casein and intact whey protein on energy expenditure and appetite regulation: a randomised, controlled, cross-over study, British journal of nutrition, 2014, vol. 112, pp. 1412-1422, abstract, p. 1413 | 1-27 |
| Y | LILLEFOSSE, Haldis H. et al., Hydrolyzed Casein Reduces Diet-induced obesity in male C57BL/6J Mice, The Journal of Nutrition, 2013, vol. 143, pp. 1367-1375, abstract, discussion | 1-27 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 3920933 B
- JP 5972235 B
- WO 2007142230 A
- WO 2003044044 A
- JP 4219707 B
- JP 2005139084 A
- JP 2009137982 A

### Non-patent literature cited in the description

- **LEVINE JA.** *J. Intern. Med.,* 2007, vol. 262 (3), 273-287
- **DULOOO AG. et al.** *Am. J. Clin. Nutr.,* 1989, vol. 49 (1), 44-50
- **NURMINEN ML et al.** *Eur. J. Clin Nutr.,* 1999, vol. 53 (11), 831-9
- **LILLEFOSSE et al.** *J. Nutrition,* 2013, vol. 143 (9), 1367-1375
- Kobunshi Kagaku No Kiso (Foundation of Polymer Science). Tokyo Kagaku Dojin, 1978, 116-119
- Tanpakushitsu/Pepuchido No Kosokuekitai Kurmatogurafi (High-Performance Liquid Chromatography of Protein/Peptide). Chemistry Extra No. 102. Kagaku Dojin, 1984, 241
- Health Promotion Act. 31 August 2009
- **TADAISHI et al.** *PLoS One,* vol. 20116 (12), e28290

SEQUENCE LISTING

<110>   Morinaga milk industry co., ltd.

<120>   Composition for promoting energy consumption

<130>   FP283-18014

<150>   JP 2017-179140
<151>   2017-09-19

<160>   15

<170>   PatentIn version 3.5

<210>   1
<211>   4
<212>   PRT
<213>   Artificial Sequence

<220>
<223>   MKP

<400>   1

Ala Met Lys Pro
1


<210>   2
<211>   4
<212>   PRT
<213>   Artificial Sequence

<220>
<223>   4-1

<400>   2

Gln Met Lys Pro
1


<210>   3
<211>   4
<212>   PRT
<213>   Artificial Sequence

<220>
<223>   4-2

<400>   3

Met Lys Pro Val
1


<210>   4
<211>   5
<212>   PRT
<213>   Artificial Sequence

<220>

```
<223>   5-1

<400>   4

Val Gln Met Lys Pro
1                   5


<210>   5
<211>   5
<212>   PRT
<213>   Artificial Sequence

<220>
<223>   5-2

<400>   5

Gln Met Lys Pro Val
1                   5


<210>   6
<211>   5
<212>   PRT
<213>   Artificial Sequence

<220>
<223>   5-3

<400>   6

Met Lys Pro Val Met
1                   5


<210>   7
<211>   6
<212>   PRT
<213>   Artificial Sequence

<220>
<223>   6-1

<400>   7

Arg Val Gln Met Lys Pro
1                   5


<210>   8
<211>   6
<212>   PRT
<213>   Artificial Sequence

<220>
<223>   6-2

<400>   8

Val Gln Met Lys Pro Val
1                   5
```

```
<210>   9
<211>   6
<212>   PRT
<213>   Artificial Sequence

<220>
<223>   6-3

<400>   9

Gln Met Lys Pro Val Met
1               5


<210>   10
<211>   6
<212>   PRT
<213>   Artificial Sequence

<220>
<223>   6-4

<400>   10

Met Lys Pro Val Met Gln
1               5


<210>   11
<211>   8
<212>   PRT
<213>   Artificial Sequence

<220>
<223>   7-1

<400>   11

Ile Arg Val Gln Met Lys Pro Val
1               5


<210>   12
<211>   7
<212>   PRT
<213>   Artificial Sequence

<220>
<223>   7-2

<400>   12

Arg Val Gln Met Lys Pro Val
1               5


<210>   13
<211>   7
<212>   PRT
<213>   Artificial Sequence

<220>
```

```
<223>   7-3

<400>   13

Val Gln Met Lys Pro Val Met
1                   5


<210>   14
<211>   7
<212>   PRT
<213>   Artificial Sequence

<220>
<223>   7-4

<400>   14

Gln Met Lys Pro Val Met Gln
1                   5


<210>   15
<211>   7
<212>   PRT
<213>   Artificial Sequence

<220>
<223>   7-5

<400>   15

Met Lys Pro Val Met Gln Leu
1                   5
```